# FASCICULE DE BREVET EUROPEEN

(11) **EP 0 324 675 B1**
(45) Date de publication et mention de la délivrance du brevet: **15.12.1993**
(21) Numéro de dépôt: 89400060.3
(22) Date de dépôt: 10.01.1989
(51) Int. Cl.: B01D 53/22, B01D 69/00, C10G 31/11, C07C 7/144

(54) **Procédé de séparation des constituants d'un mélange en phase gazeuse au moyen d'une membrane composite**
Verfahren zur Trennung der Komponenten eines Gasgemisches mittels einer zusammengesetzten Membran
Process for separating the components of a gaseous mixture using a composite membrane

(30) Priorité: 11.01.1988 FR 8800243
(43) Date de publication de la demande: 19.07.1989
(73) Titulaire: INSTITUT FRANCAIS DU PETROLE, 92502 Rueil-Malmaison (FR)
(72) Inventeur: Rojey, Alexandre, F-92380 Garches (FR); Deschamps, André, F-78590 Noisy le Roi (FR); Grehier, Alain, F-75012 Paris (FR); Robert, Eric, F-92500 Rueil Malmaison (FR)

(56) Documents cités:
- EP-A- 0 154 248
- EP-A- 0 254 758
- FR-A- 2 011 125
- FR-A- 2 079 460
- US-A- 2 924 630
- US-A- 3 567 666
- US-A- 4 740 219
- CHEMICAL ABSTRACTS, vol. 85, no. 1, 05 juillet 1976, Columbus, OH (US); F. WOLF et al., p. 410, no. 5102z#
- JOURNAL OF MEMBRANE SCIENCE, vol. 35, no. 1, 15 décembre 1987, Elsevier Science Publishers B.V., Amsterdam (NL); H.J.C. TE HENNEPE et al., pp. 39-55#

## Description

L'invention concerne un procédé de séparation des constituants d'un mélange en phase gazeuse au moyen d'une membrane composite comportant un adsorbant et notamment d'un mélange comprenant des hydrocarbures.

Les procédés de séparation par membranes et notamment les procédés de perméation gazeuse ont connu récemment un développement important grâce à la mise au point de membranes asymétriques dont la couche active a une très faible épaisseur et de modules à fibres creuses de très grande surface spécifique.

Toutefois ces procédés qui trouvent dès à présent des applications commerciales, par exemple pour la séparation d'hydrogène d'un mélange gazeux d'hydrogène et d'hydrocarbures ou la séparation de gaz carbonique d'un mélange gazeux de gaz carbonique et d'hydrocarbures, n'ont pas encore abouti dans le domaine de la séparation des isomères d'hydrocarbures.

En effet, les membranes à base de polymères qui ont été développées jusqu'à présent s'avèrent d'une sélectivité insuffisante pour séparer des isomères tels que paraffines normales/isoparaffines, meta et paraxylène, oléfines/paraffines.

Il a été découvert que la séparation des isomères d'hydrocarbures devient possible si l'on met en oeuvre une membrane de conception nouvelle.

En outre le procédé selon l'invention permet d'effectuer dans des conditions plus avantageuses des séparations de mélanges gazeux tels que hydrogène/méthane et méthane/CO₂.

La membrane utilisée dans le procédé selon l'invention est une membrane composite incorporant une phase adsorbante sélective vis à vis d'un des constituants présents dans le mélange à séparer.

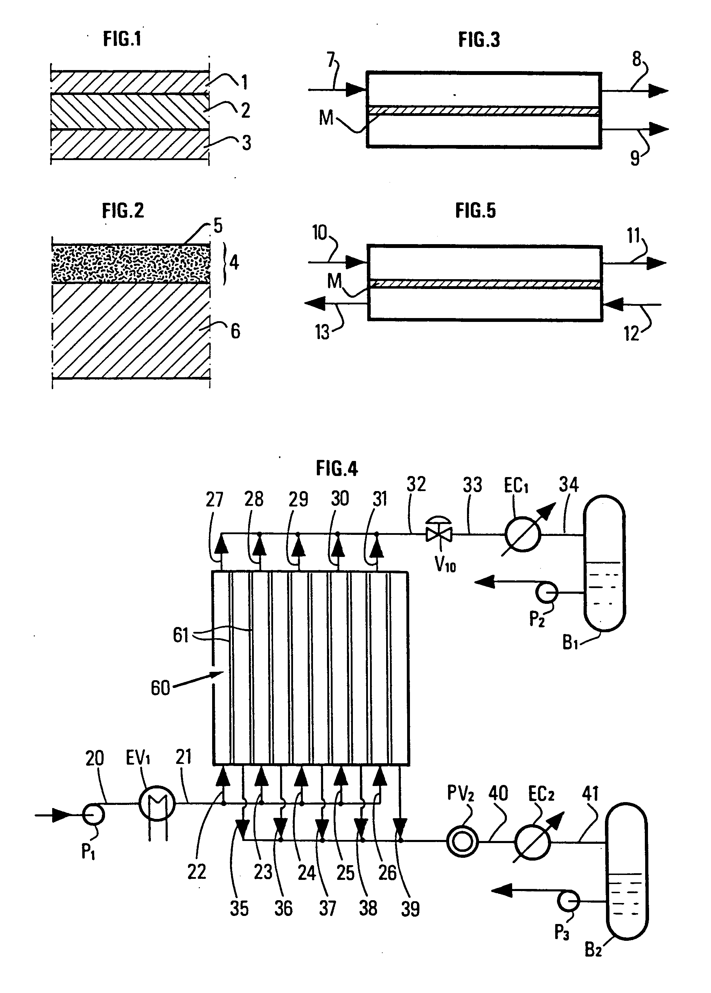

Les membranes composites connues selon l'art antérieur font intervenir une superposition de couches parallèles formées de matériaux de propriétés différentes selon le schéma de la figure 1, sur lequel on a figuré de telles couches parallèles 1, 2, 3.

Par exemple il est indiqué dans le brevet US-A-4 230 463 comment une couche de silicone très perméable et peu sélective peut être superposée à une couche active en polysulfone pour en améliorer les performances, en permettant de réduire l'effet de microfissures dans la couche active de polysulfone.

Selon ce principe, il a déjà été proposé dans le brevet européen EP-A-0180200 d'utiliser une membrane composite comprenant une couche active formée par un matériau tel qu'une zéolithe.

En pratique, il est toutefois difficile de réaliser une couche cristalline d'un matériau tel qu'une zéolithe qui soit suffisamment fine et régulière.

Le brevet européen EP-A-0180200 préconise pour y arriver de réaliser une couche sélective par imprégnation, en piègeant des particules de zéolithe dans les pores d'un milieu poreux, les pores devant être suffisamment bien calibrés et les particules de dimension relativement uniforme. C'est le cas aussi du brevet US-A-3567666 qui décrit une structure à base de polymère, poreuse, dont les canaux contiennent des particules microporeuses de tamis moléculaire, les pores de ces particules étant interconnectés par les canaux.

Ces méthodes se heurtent à de nombreuses difficultés : difficulté de calibrer les pores du support poreux et les particules de zéolithe, de contrôler l'épaisseur du film de zéolithe, de réaliser une couche continue.

D'autre part, il peut être difficile de réaliser des traitements après piègeage des particules, notamment si le support poreux est à base de polymère et si le traitement nécessite une température élevée.

Le brevet FR-A-2079460 décrit une membrane constituée a) d'un polymère, b) d'une zéolithe dispersée dans le polymère et c) d'un support de renforcement tel qu'un polyester ou un polyamide. L'épaisseur des membranes ainsi obtenues est comprise entre 100 et 1000 micromètres. De ce fait, la diffusion du produit devant traverser la membrane est faible et les débits de produits sont réduits. On est alors amené à augmenter la différence de pression entre les faces de la membrane pour récupérer un débit de sortie acceptable. Mais cela se traduit par un coût d'investissement plus élevé en matériel, une consommation d'énergie également coûteux et par un risque accru de rupture de la membrane.

Il est également connu selon le brevet EP-A-0254758 et le document "Journal of Membrane Science vol. 35, n° 1, 15 dec. 1987, pages 39-55 Elsevier Science Publishers B.V. Amsterdam" une membrane comprenant une matrice polymérique constitué d'un matériau élastomère à base de silicone (silicone rubber) ou de polyisoprène, où est dispersé un matériau zéolithique.

De plus, il est connu selon le brevet EP-A-0154248 une membrane comprenant des groupements fonctionnels ou des zéolithes greffés chimiquement sur des molécules de polymère.

En outre, l'art antérieur est illustré par le brevet US-A-2924630 décrivant un procédé de séparation par des membranes contenant des zéolithes dont les diamètres des pores sont comprises entre 3 et 15 Angströms et par le document Chem. Abstract Volume 85, 1976 n° 1, page 410, n° 5102Z qui décrit une membrane à tamis moléculaire utilisée pour la séparation du méthane et des oléfines inférieurs.

Enfin, l'art antérieur est illustré par le document Patent Abstract of Japan volume 9, n° 317 (2040), 12 dec. 1985 qui décrit une membrane avec de la silice dont la surface est traitée par un agent de liaison et par le brevet FR-A-2011125 où la membrane comprend une seule couche de granules noyés en partie dans une feuille de matière plastique.

Le principe de réalisation des membranes utilisées dans le procédé selon l'invention est différent et il sera mieux compris à la vue des figures suivantes parmi lesquelles :
- la figure 1, comme indiqué ci-haut, représente une membrane composite selon l'art antérieur ;
- la figure 2 illustre le principe de réalisation des membranes utilisées dans le procédé selon l'invention ;
- la figure 3 illustre le procédé de séparation selon l'invention ;
- la figure 4 montre un schéma de séparation d'un mélange de pentane normal et d'isopentane ;
- la figure 5 représente le procédé de séparation selon l'invention, où la pression partielle du constituant devant traverser la membrane est abaissée par circulation d'un éluant gazeux du côté où l'on soutire ce constituant ;
- la figure 6 montre un couplage d'un procédé d'isomérisation combiné à un procédé de séparation.

Il a été découvert qu'en dispersant des particules d'une phase adsorbante sélective dans une phase polymère continue et non poreuse et à condition de respecter des conditions qui sont précisées par la suite, il est possible de réaliser une couche sélective composite sans avoir les inconvénients précités et avec une efficacité améliorée.

Un autre objet de l'invention est relatif au procédé d'obtention d'une membrane asymétrique de très faible épaisseur et à la membrane ainsi obtenue.

Un autre objet concerne un procédé d'enrichissement en iso-alcanes combinant une étape d'isomérisation d'une coupe de n-alcanes suivie d'une étape de séparation des n- et iso- alcanes.

La membrane utilisée dans le procédé selon l'invention (figure 2) est ainsi composée d'au moins une couche active comprenant la phase polymère continue (4) et des particules (5) de phase adsorbante dispersée et d'une couche poreuse support (6).

Dans la couche active, les particules de phase adsorbante sélective sont dispersées de manière sensiblement uniforme et homogène sur toute l'épaisseur de la couche sélective et non implantées à la surface. La phase continue forme un film dense dépourvu de structure poreuse à l'échelle des particules.

Le transfert d'un constituant à travers la couche sélective implique nécessairement la perméation dudit constituant à travers la phase continue par un mécanisme de sorption dans le matériau formant la phase continue puis de diffusion dans ledit matériau. La présence de la phase dispersée peut contribuer soit à accélérer soit à freiner le transfert d un constituant à travers la couche sélective selon que la perméation dudit constituant à travers la phase dispersée est plus rapide ou moins rapide qu'à travers la phase continue.

Il a été ainsi découvert qu'il est possible de réaliser une couche active perméable et sélective en associant, dans des conditions qui font l'objet de la description plus détaillée du procédé, une phase dispersée sélective à une phase continue perméable mais non sélective.

Le procédé selon l'invention comprend les étapes suivantes : (a) on met en contact un mélange d'au moins deux constituants A et B en phase gazeuse ou vapeur avec une première face d'une membrane composée (i) d'au moins une couche active comprenant une phase adsorbante solide, sélective vis à vis du constituant A, dispersée de manière sensiblement uniforme dans un polymère non élastomérique, non poreux et constituant une phase continue et (ii) d'une couche poreuse servant de support, (b) on maintient une pression partielle de constituant A plus faible sur la seconde face de la membrane que sur la première face de la membrane et on recueille sur la seconde face de ladite membrane, sans changement de phase, un mélange enrichi en constituant A et appauvri en constituant B, et (c) on recueille sur la première face de la membrane un mélange enrichi en constituant B et appauvri en constituant A. De préférence les étapes (a), (b) et (c) sont opérées de manière continue.

Par polymère non poreux, on entend un polymère à travers lequel la migration des produits s'effectue sensiblement par dissolution et diffusion moléculaire. On exclut de ce fait un transport dans le polymère entre les particules de zéolithe pouvant être régi par une loi de type Knudsen.

De manière plus précise, l'épaisseur de la couche active est inférieure à 1 micromètre. Elle est alors suffisamment perméable vis-à-vis du constituant A. La cinétique de transfert est particulièrement améliorée, notamment avec une épaisseur de 0,1 à 0,5 micromètre.

La dimension moyenne des particules constituant la phase adsorbante dispersée est généralement inférieure à 25 micromètres, avantageusement inférieure à 7,5 micromètres. Avec une taille moyenne préférée de particules inférieure à 0,1 micromètre, par exemple 200 à 900 Angströms (1 Angström = 1X10⁻¹⁰m), on a obtenu d'excellents résultats de séparation et plus particulièrement avec une taille moyenne de particules inférieure à 500 Angströms.

Le procédé selon l'invention présente l'avantage d'une meilleure efficacité de séparation et d'une mise en oeuvre facilitée.

Pour l'obtention de particules dont la dimension est supérieure à environ 1 micromètre, on a recours par exemple aux moyens classiques tels que broyage et tamisage. Par contre, pour obtenir des particules de dimension inférieure à environ 1 micromètre, on modifie par exemple les conditions de synthèse de la zéolithe concernée de telle manière que l'on favorise par exemple le processus de nucléation par rapport au processus de croissance des cristallites. On peut par exemple à cette fin abaisser la température de synthèse ou opérer en sursaturation des composants ou encore limiter le temps de réaction. On peut aussi opérer par ultrafiltration.

L'épaisseur de la couche active peut être contrôlée de la façon suivante :
- Pour des épaisseurs par exemple de 1,0 micromètre, on peut utiliser des moyens de mesure macroscopiques tels qu'un palmer ou un comparateur.
- Pour des épaisseurs comprises par exemple entre 0,1 et 1 micromètres, on peut utiliser la technique de micrographie ou bien cette épaisseur peut être déduite de la mesure du débit d'un gaz défini tel que l'hydrogène ou l'hélium à travers une surface de membrane connue pour une différence de pression régnant de part et d'autre de la membrane déterminée et de la mesure de la perméabilité d'une membrane de même nature et d'épaisseur suffisante pour être mesurée par des moyens conventionnels tels que décrits ci-dessus.

Le principe du procédé selon l'invention est illustré par le schéma de la figure 3.

Un mélange comprenant au moins deux constituants A et B à séparer arrive par le conduit 7 et circule d'un côté de la membrane M réalisée selon le schéma de principe de la figure 2.

Le mélange se trouve en phase gazeuse ou vapeur et le constituant A passe sélectivement à travers la membrane M sous l'effet d'une différence de pression partielle.

Le mélange enrichi en constituant A est évacué par le conduit 9 et le mélange appauvri en constituant A et donc enrichi en constituant B est évacué par le conduit 8.

Pour que le procédé selon l'invention puisse être opéré, il est nécessaire, comme cela a été indiqué, de respecter certaines conditions.

De manière à pouvoir réaliser une couche active suffisamment homogène, la dimension des particules de phase dispersée doit être suffisamment petite par rapport à l'épaisseur de la couche active, le rapport de la taille moyenne des particules de phase dispersée sur l'épaisseur de la couche active devant être inférieur à 0,25, par exemple 0,01 à 0,25.

De telles particules très fines peuvent être obtenues par exemple par ultrafiltration à partir d'une suspension de cristaux.

Le rapport de la masse de phase adsorbante dispersée à la masse de phase polymère continue doit être suffisamment élevé et supérieur à 1, par exemple 1/1 à 10/1, et de préférence 2/1 à 6/1.

Du fait de son épaisseur très faible la couche active doit être déposée sur au moins une couche poreuse à laquelle elle adhère, de résistance mécanique suffisante.

Cette couche support, pour être suffisamment perméable, est en général macroporeuse, c'est-à-dire présenter des pores dont le diamètre est à l'échelle des particules de phase dispersée, soit par exemple supérieur à 25 micromètres.

Elle peut être constituée, soit par un matériau poreux, soit par un autre matériau, par exemple un métal fritté ou une mécanique frittée.

La phase adsorbante doit être sélective vis-à-vis du constituant A.

Les tamis moléculaires formés par différentes zéolithes naturelles ou de synthèse sont déjà utilisés pour réaliser des séparations sélectives entre des constituants qui diffèrent par la taille des molécules ou par leur affinité chimique. Toutefois, leur mise en oeuvre en lit fixe nécessite une opération discontinue et un jeu complexe de vannes, ce que permet d'éviter le procédé selon l'invention.

Les différents types de tamis moléculaires peuvent être utilisés pour former la phase dispersée dans le polymère non poreux de la membrane utilisé dans le procédé selon l'invention.

Ainsi, par exemple :
La zéolithe de type KA dite aussi tamis moléculaire 3 A permet de séparer un constituant dont la molécule a une taille inférieure à 3 Angströms (1 Angström = 1X10⁻¹⁰ mètre) d'un constituant dont la molécule a une taille supérieure à 3 Angströms. Elle permet ainsi par exemple d'effectuer une séparation entre l'eau qui passe à travers les pores de la zéolithe et le méthane qui est exclu.

La zéolithe de type NaA dite aussi tamis moléculaire 4 A permet de séparer un constituant dont la molécule a une taille inférieure à 4 Angströms d'un constituant dont la molécule a une taille supérieure à 4 Angströms.

La zéolithe de type CaA dite aussi tamis moléculaire 5 A permet de séparer les paraffines normales d'un mélange de paraffines normales et d'isoparaffines.

Les zéolithes K-BaY, Sr-BaX, K-BaX et ZSM5 permettent de séparer le paraxylène d'un mélange de paraxylène, métaxylène, orthoxylène et éthylbenzène. Les zéolithes NaY et Sr-KX permettent de séparer l'éthylbenzène d'un mélange d'éthylbenzène, orthoxylène, métaxylène et paraxylène.

Des zéolithes telles que les zéolithes CaX et SrX permettent de séparer les oléfines d'un mélange d'oléfines et de paraffines.

Les zéolithes sont donc particulièrement bien adaptées pour former la phase dispersée dans la couche sélective de la membrane utilisée dans le procédé selon l'invention.

Toutefois d'autres matériaux peuvent être également utilisés. Par exemple, on sait réaliser des tamis moléculaires à base de carbone, présentant des pores de taille calibrée, qui peuvent être également utilisés comme phase dispersée. Des particules divisées de charbon actif peuvent être également utilisées.

On peut dans certains cas également utiliser des phases adsorbantes sélectives qui ne sont pas des tamis moléculaires telles que par exemple certaines résines échangeuses d'ions.

Les matériaux pouvant être utilisés pour constituer la couche active seront des polymères non élastomériques de type thermoplastique ou thermodurcissable. Parmi les polymères techniques habituels on pourra de préférence utiliser des matériaux chargés ou non, performants à hautes températures. A titre d'exemples nous pouvons citer : parmi les thermoplastiques :
a) la famille des polysulfones (polysulfure de phénylène, polyarylsulfone, polyéthersulfone),
b) la famille des cétones (polyétherethercetone, polyéthercetone)
c) les polyétherimides,
d) les polyamide-imides,
e) les résines ionomères,
f) les polyimides thermoplastiques,
g) les polymères fluorés.

Parmi les thermodurcissables outre les traditionnelles résines polyesters ou époxides, on peut utiliser les résines silicones et polyimides.

On peut aussi utiliser des copolymères.

De manière préférée, ce sont les polyimides qui sont utilisés. On peut alors opérer à des températures relativement élevées, par exemple, au-dessus de 100° C, ce qui permet d'améliorer la cinétique de transfert à travers la membrane.

Concernant la couche poreuse support, on peut utiliser de préférence des polymères de même nature que ceux constituant la couche active. On peut aussi utiliser des métaux frittés tels que des aciers inoxydables, du nickel ou des céramiques frittées.

Le diamètre des pores de cette couche support est généralement compris entre 3 et 100 micromètres.

Pour obtenir une couche active de membrane ayant une épaisseur définie selon l'invention, on peut par exemple notamment pour l'obtention d'une couche active d'épaisseur inférieure à 1 micromètre, faire coaguler, par contact durant une période généralement comprise entre 1 et 24 heures et à une température habituellement comprise entre - 20° C et + 100° C entre un antisolvant et l'une des faces d'un film constitué d'un polymère non élastomérique en solution dans un solvant contenant la phase adsorbante dispersée, une couche active qui constitue la couche sélective de la membrane. La température est généralement choisie en fonction du solvant du polymère. On obtient ainsi sur cette face du film la couche active comprenant la phase adsorbante dispersée dans une couche de polymère non poreux et une couche de polymère poreux sous-jacente indissociée de la couche active, qui constitue la couche support.

Par indissociée, on entend que la couche active et la couche support sont solidaires et ne se différencient que par leurs structures respectives, le polymère constituant les deux couches étant le même polymère mais ledit polymère ayant une structure continue dans la couche active et une structure poreuse dans la couche support, les deux couches contenant la phase adsorbante dispersée de manière sensiblement homogène dans le polymère.

Selon une autre caractéristique du procédé de préparation de la membrane, le film de polymère peut être déposé sur un support, de préférence poreux, par exemple un métal fritté.

Différents solvants peuvent être utilisés pour solubiliser le polymère, soit des solvants légers, de température d'ébullition relativement basse tels que par exemple le dichlorométhane, soit des solvants lourds de température d'ébullition relativement élevée tels que par exemple la N-méthylpyrrolidone.

L'agent coagulant qui permet de précipiter le polymère en formant une couche active de très faible épaisseur à la surface joue le rôle d'un anti-solvant vis à vis du polymère et peut être par exemple de l'eau ou encore un solvant polaire organique tel que par exemple l'acétone.

Des mélanges plus complexes peuvent être également utilisés et les différentes méthodes connues de l'homme de l'art pour réaliser une membrane asymétrique à partir d'un matériau polymère peuvent être employées, la condition essentielle pour réaliser une membrane selon le procédé étant de mettre en suspension dans la solution de polymère des particules de phase dispersée en respectant les conditions indiquées.

La dispersion des particules de zéolithe et la stabilité de la suspension peuvent être améliorées par l'adjonction d'agent tensioactif soluble dans le solvant du polymère.

On peut également effectuer un prétraitement de la phase dispersée par un agent de liaison tel que par exemple un silane avant de la mélanger à la solution de polymère. Selon un autre mode de réalisation l'agent de liaison peut être incorporé dans la solution de polymère avant l'introduction de la phase dispersée. Ces deux modes de réalisation permettent d'introduire l'agent de liaison à l'interface entre la phase dispersée et la phase continue et d'assurer ainsi une meilleure adhésion entre ces deux phases.

Le procédé selon l'invention permet d'améliorer très sensiblement les performances des séparations de mélanges gazeux par perméation gazeuse, chaque fois que l'adsorbant utilisé laisse passer préférentiellement un des constituants présents dans le mélange.

Les membranes sont généralement mises en oeuvre sous forme de plaques planes.

D'autres géométries peuvent être également envisagées. Il est possible par exemple d'enrouler les membranes selon une spirale afin de réaliser des dispositifs de séparation plus compacts.

Il peut être également avantageux de mettre les membranes sous forme de fibres creuses de diamètre inférieur à 5 mm pour augmenter la surface spécifique d'échange. Ces fibres creuses sont obtenues par extrusion à travers l'orifice d'une filière, puis immersion de la fibre creuse dans un bain d'antisolvant. On obtient ainsi une couche sélective de très faible épaisseur supportée par une structure macroporeuse.

Le transfert de constituant A à travers la membrane peut être opéré dans des conditions sensiblement isothermes.

La température à laquelle ce transfert est opéré se situe de préférence entre 30 et 150 °C.

Les exemples suivants illustrent l'invention.

### EXEMPLE 1 (comparatif)

a) On porte 12,5 parties en poids de zéolithe 4 A constituée de particules dont la taille moyenne est inférieure à 1 micromètre à 350° C pendant 24 heures sous un vide de 0,13 Pa (10⁻³ torr).
   Après refroidissement sous vide, on ajoute sous vide 100 parties en poids de n-méthylpyrrolidone (NMP). Le mélange zéolithe 4 A - NMP remis à pression atmosphérique constitue la suspension B.
   On prépare la solution A en mettant en solution dans 45 parties en poids de NMP, 11 parties en poids de polyetherimide de marque ULTEM.
   Après agitation, la suspension B est mélangée à la solution A.
   Le mélange est ensuite porté sous azote à 130° C jusqu'à évaporation d'environ la moitié de la NMP afin d'obtenir un produit de consistance visqueuse.
b) Le mélange est enduit sur un fritté d'acier inoxydable de classe 05 d'épaisseur égale à 1 mm de manière uniforme pour avoir un film d'épaisseur égal à 70 micromètres après évaporation du solvant.
   Cette évaporation du solvant est obtenue par chauffage progressif à pression atmosphérique de 50 à 200° C suivi d'un séjour de 24 heures à 200° C et passage sous vide à cette température pendant 24 heures.
c) Séparation. Par le conduit 7 (figure 3), on envoie, avec un débit de 1 m³/h mesuré dans les conditions normales de température et de pression, un mélange équimolaire d'hydrogène et de méthane sous une pression de 60 bar. Les compartiments à haute et basse pression (ΔP = 30 bar) sont séparés par la membrane ci-dessus de 1 m² de surface. Ils sont maintenus à une température voisine de l'ambiante. Par le conduit 9, on évacue 9 g/h d'hydrogène et 0,003 g/h de méthane.

### EXEMPLE 2

On réalise l'exemple 2 selon l'étape la de l'exemple 1 sauf que les particules utilisées ont une taille moyenne inférieure à 500 Angströms (1 Angström = 1X10⁻¹⁰m).
Le mélange visqueux est enduit sous azote sur une plaque de verre à l'aide d'un racleur de manière à obtenir un film uniforme de 100 micromètres environ d'épaisseur. Après 10 minutes sous azote, l'ensemble plaque de verre - film est plongé dans un bain d'eau distillée servant d'agent coagulant, à température ambiante où il est maintenu pendant 16 heures.
L'épaisseur de la couche active ainsi obtenue déduite des mesures de débit de gaz (He) et de perméabilité est estimée à 0,8 micromètre.
Après séparation, selon l'exemple 1c, par la membrane ainsi obtenue, on évacue 1020 g/h d'hydrogène et 0,32 g/h de méthane.
On observe dans ce cas qu'avec la même surface de membrane, il est possible de produire un débit d'hydrogène purifié beaucoup plus important, tout en laissant passer moins de méthane.

### EXEMPLE 3

Le même procédé peut être utilisé pour séparer du méthane et de l'eau en phase gazeuse, en utilisant une membrane à base de polymère, par exemple en polyétherimide, et un tamis moléculaire de type 3 A qui laisse passer l'eau et arrête le méthane, dans les mêmes conditions que celles définies dans l'exemple 2.

Il est possible ainsi de déshydrater un gaz naturel jusqu'à des teneurs en eau très faibles, par exemple moins de 5 ppm en volume, à condition de tirer au vide du côté de la membrane où l'on soutire l'eau afin de maintenir une pression partielle d'eau du côté où l'eau est soutirée inférieure à la pression partielle d'eau dans le gaz du côté où le gaz déshydraté est soutiré.

### EXEMPLE 4 (comparatif)

On réalise un mélange équimolaire de pentane normal et d'isopentane.

Par la pompe P1 (Figure 4), on prélève 190,4 g/h du mélange en phase liquide et on l'envoie par le conduit 20 dans l'échangeur EV1, d'où il ressort en phase vapeur à la température de 100 °C. Cette phase vapeur (21) est envoyée par les conduits 22 à 26 à un perméateur 60 à plaques planes 61. Afin de simplifier le dessin, le nombre exact de plaques n'a pas été représenté. On opère avec 100 plaques de 1,6 m² chacune. Chaque plaque est constituée par une membrane constituée d'un ensemble couche active sur un support poreux en métal fritté. La membrane a été obtenue en étalant sur un support poreux en métal fritté de 5 mm d'épaisseur, un film constitué par une suspension de tamis moléculaire 5 A dont les cristallites ont une dimension moyenne de 0,1 µm, dans une solution de polyétherimide de type ULTEM dans du dichloroéthane, le rapport en masse du tamis sur la masse totale de tamis et de polyétherimide étant de 0,8. Après évaporation de solvant on obtient un film d'une épaisseur d'environ 10 micromètres.

Par les conduits 27 à 31, on soutire une phase vapeur enrichie en isopentane qui a comme composition molaire :
iC₅ : 0,987
nC₅ : 0,013
Le mélange gazeux ainsi obtenu est évacué par le conduit 32 et passe à travers la vanne de détente V10 qui permet de maintenir dans les conduites 27 à 31 une pression contrôlée et constante de 4,5 bars. Le mélange détendu est envoyé par le conduit 33 au condenseur EC1, d'où il ressort par le conduit 34, le mélange en phase liquide étant recueilli dans le ballon B1, d'où il est évacué par la pompe P2.

Par les conduites 35 à 39, on soutire un débit total de 98,7 g/h d'une phase vapeur enrichie en pentane normal, qui a comme composition molaire :
nC₅ : 0,952
iC₅ : 0,048
La pompe à vide PV2 permet de maintenir dans les conduits 35 à 39 une pression de 0,6kPa. A la sortie de la pompe à vide PV2, le mélange vapeur est envoyé par le conduit 40 au condenseur EC2 d'où il ressort par le conduit 41 ; le mélange en phase liquide est recueilli dans le ballon B2, d'où il est évacué par la pompe P3.

### EXEMPLE 5

On opère avec le même mélange que dans l'exemple 4 et le même dispositif constitué de 100 plaques de 1,6 m² représenté sur le schéma de la figure 4.

Dans ce cas toutefois, l'élaboration de la membrane a été modifiée et la technique employée consiste à réaliser une membrane composite asymétrique.

Pour obtenir une telle membrane plane, on a réalisé une solution de polyétherimide commercialisé sous la marque ULTEM dans un mélange renfermant 80 % en poids de dichlorométhane, 5 % en poids de trichloroéthane et 10 % en poids de xylène. La solution contient 25 % en poids de polyétherimide. Cette solution a été chargée en particules de tamis 5A d'une taille moyenne inférieure à 250 Angströms (1 Angström = 10⁻¹⁰ m), de manière à obtenir une suspension renfermant 50 % en poids de tamis.

La solution de polymère ainsi chargée est ensuite étalée au moyen d'un applicateur sur une plaque plane en métal fritté.
On réalise une évaporation partielle du solvant pendant 15 secondes à une température de 20 °C sous balayage d'azote sec. La coagulation du polymère est alors réalisée en mettant en contact le film de polymère chargé avec de l'acétone à 20 °C pendant une heure. La plaque est alors séchée en étuve à 100 °C pendant 4 heures, puis soumise à un traitement thermique à 180 °C pendant 4 heures sous vide. L'épaisseur de la couche active est alors selon l'invention.

On constate qu'en utilisant le même dispositif que dans l'exemple 4, il est possible dans ce cas d'augmenter considérablement le débit qui transfère à travers la membrane.

Après adaptation des pompes P1, P2, P3 et des échangeurs EV1, EC1 et EC2 ainsi que de la pompe à vide PV2, on envoie par la pompe P1 un débit de 17,2 kg/h de mélange en phase liquide. Après vaporisation dans l'échangeur EV1, cette phase vapeur est envoyée par les conduits 22 à 26 au perméateur.

Par les conduits 27 à 31 on soutire un débit total de 8,3 kg/h d'une phase vapeur enrichie en isopentane qui a comme composition molaire :
iC₅ : 0,988
nC₅ : 0,012
Le mélange gazeux est maintenu à une pression contrôlée de 4,5 bars au niveau des conduits 27 à 31 au moyen de la vanne de détente V10. A la sortie de la vanne de détente V10, le mélange est condensé dans le condenseur EC1 et évacué par la pompe P2.

Par les conduits 35 à 39 on soutire un débit total de 8,9 kg/h d'une phase vapeur enrichie en pentane normal qui a comme composition molaire :
nC₅ : 0,955
iC₅ : 0,045
La pompe à vide PV2 permet, comme dans l'exemple 1, de maintenir au niveau des conduits 35 à 39 une pression de 0,6 kPa.

Dans les exemples 4 et 5, la différence entre les pressions partielles d'équilibre du constituant A de part et d'autre de la membrane est réalisée en tirant au vide du côté de la membrane où l'on soutire le constituant A.

Il est également possible de réaliser une telle différence entre les pressions partielles d'équilibre en faisant circuler un éluant du côté de la membrane où l'on soutire le constituant A.

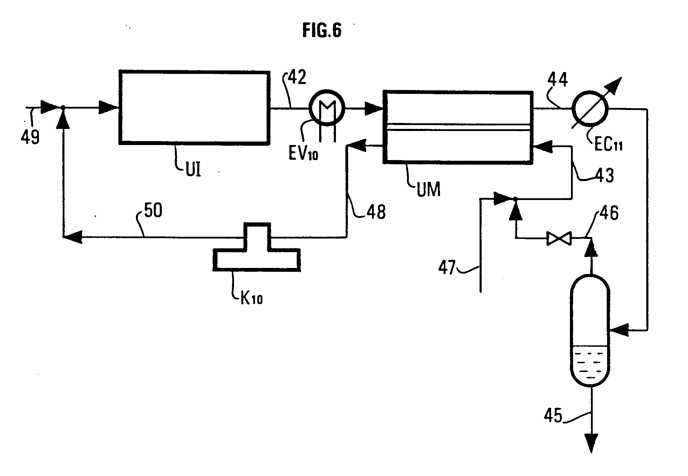

On peut opérer dans ce cas selon le schéma illustré sur la figure 5.

Le mélange à séparer arrive en phase gazeuse par le conduit 10. Par le conduit 12 on fait arriver un éluant gazeux, ce qui permet d'abaisser la pression partielle d'équilibre en constituant A et de transférer le constituant A à travers la membrane.

L'éluant peut être constitué par exemple par de l'azote ou de l'hydrogène.

Ainsi par exemple dans le cas de la séparation d'un mélange de paraffines normales et d'isoparaffines, il peut être particulièrement avantageux d'utiliser de l'hydrogène comme éluant lorsque le procédé de séparation est couplé à un procédé d'isomérisation opérant en présence d'hydrogène.

On peut dans ce cas opérer par exemple selon la disposition qui est illustrée par le schéma de la figure 6.

Un mélange équimolaire d'isopentane et de normal-pentane est admis sous une pression de 2 MPa (20 bar) à la température de 250° C en phase gazeuse par la ligne 49 dans l'unité d'isomérisation UI en mélange avec un courant de recyclage renfermant 90 % en volume d'hydrogène et 10 % en volume de normal pentane arrivant par la ligne 50.

Le mélange qui sort en phase gazeuse par le conduit 42 de l'unité d'isomérisation UI à la température de 280° C contient une fraction de normal-pentane exprimée en masse par rapport à la masse totale de normal-pentane et d'isopentane égale à 20 %. Le mélange est ensuite refroidi dans l'échangeur EV 10, d'où il est envoyé, toujours en phase gazeuse, à un dispositif de séparation UM équipé de membranes composites asymétriques réalisées selon la procédure décrite dans l'exemple 5 : le mélange enrichi en normal-pentane qui a traversé la membrane est élué par de l'hydrogène qui arrive par les conduits 47 et 43. Le mélange enrichi en isopentane qui sort par le conduit 44 du dispositif de séparation UM contient de l'hydrogène qui a transféré à travers la membrane et un mélange de normal-pentane et d'isopentane contenant environ 2 % de normal-pentane par rapport à la masse totale de normal-pentane et d'isopentane. Après refroidissement à environ 30° C dans l'échangeur EC 11 la fraction du mélange riche en isopentane est condensée puis évacuée par la conduite 45. La phase gazeuse (46) est jointe au courant d'hydrogène (47). Le mélange d'hydrogène et de normal-pentane (48) est comprimé (K 10) et recyclé à l'unité d'isomérisation.

## Revendications

1. Procédé de séparation d'un mélange d'au moins deux constituants A et B, dans lequel (a) on met en contact ledit mélange en phase gazeuse ou vapeur avec une première face d'une membrane composée (i) d'au moins une couche active comprenant une phase adsorbante solide, sélective vis à vis du constituant A, dispersée dans un polymère non poreux, non élastomérique et constituant une phase continue et (ii) d'une couche poreuse servant de support adhérant directement à ladite couche active, le rapport de la masse de phase dispersée sur la masse de phase continue dans la couche active de la membrane étant supérieur à 1, (b) on maintient une pression partielle de constituant A plus faible sur la seconde face de la membrane que sur la première face de la membrane et on recueille sur la seconde face de ladite membrane, sans changement de phase, un mélange enrichi en constituant A et appauvri en constituant B, et (c) on recueille sur la première face de la membrane un mélange enrichi en constituant B et appauvri en constituant A, le procédé étant caractérisé en ce que l'épaisseur de la couche active est inférieure à 1 micromètre et en ce que le rapport de la taille moyenne des particules de phase adsorbante dispersée sur l'épaisseur de la couche active est inférieure à 0,25.

2. Procédé selon la revendication 1, caractérisé en ce que la phase adsorbante dispersée dans le polymère en phase continue est constituée par une zéolithe de type tamis moléculaire.

3. Procédé selon l'une des revendications 1 à 2 dans lequel la taille moyenne des particules est inférieure à 0,1 micromètre.

4. Procédé selon l'une des revendications 1 à 3 dans lequel la membrane est une membrane asymétrique obtenue par mise en contact d'un antisolvant sur la première face d'un film de polymère en solution dans un solvant comprenant la phase adsorbante dispersée dans des conditions telles que l'on fait coaguler ladite couche active s'appuyant de manière indissociée sur le film sous-jacent devenu poreux qui constitue la couche support.

5. Procédé selon l'une des revendications 1 à 4, caractérisé en ce que le constituant A est de l'hydrogène et le constituant B du méthane, et en ce que la phase adsorbante dispersée est constituée par un tamis moléculaire de type 4A.

6. Procédé selon l'une des revendications 1 à 5, caractérisé en ce que le constituant A est du méthane et le constituant B de l'eau, et en ce que la phase adsorbante dispersée est constituée par un tamis moléculaire de type 3A.

7. Procédé selon l'une des revendications 1 à 6 dans lequel le mélange comprend des n-paraffines et dans lequel la couche active comprend un tamis moléculaire de type 5 A, caractérisé en ce qu'on effectue la combinaison suivante :
a) on envoie en phase vapeur de l'hydrogène et le mélange dans une zone d'isomérisation dans des conditions d'isomérisation telles que l'on recueille des n-paraffines, des isoparaffines et de l'hydrogène ;
b) on met en contact en phase vapeur le produit ainsi recueilli dans une première partie d'une zone de séparation en amont de la membrane et on effectue la séparation des n-paraffines, des iso-paraffines et de l'hydrogène en faisant circuler dans une seconde partie de ladite zone en aval de la membrane au moins en partie de l'hydrogène résultant de l'étape c) ci-dessous ;
c) on récupère en sortie de ladite première partie un second mélange d'hydrogène et d'isoparaffines, on condense ce second mélange et on recueille d'une part une fraction ne contenant sensiblement que des isoparaffines et d'autre part de l'hydrogène que l'on recycle selon l'étape b) dans la seconde partie de la zone de séparation ;
d) on récupère en sortie de la seconde partie de la zone de séparation un troisième mélange enrichi en n-paraffines contenant de l'hydrogène ; et
e) on recycle au moins en partie le troisième mélange dans la zone d'isomérisation, selon l'étape a).

8. Procédé selon l'une des revendications 1 à 7 dans lequel le polymère non élastomérique est un polyimide.

9. Procédé selon l'une des revendications 1 à 8 dans lequel le rapport de la masse de phase dispersée sur la masse de phase continue dans la couche active de la membrane est de 2 : 1 à 6 : 1.

## Claims

1. Process for separation of a mixture of at least two constituents A and B in which (a) said mixture in the gas or vapor phase is contacted with the first face of a membrane composed of (i) at least one active layer comprising a solid adsorbent phase, selective vis-a-vis constituent A, dispersed in a non porous non elastomeric polymer and constituting a continuous phase and (ii) a porous phase serving as a support which directly adheres to said active layer, the ratio of mass of dispersed phase to the mass of the continuous phase in the active layer of the membrane being greater than 1, (b) the partial pressure of constituent A is kept lower at the second face of the membrane than at the first face and a mixture enriched in constituent A and impoverished in constituent B is collected on the second face of said membrane without a change in phase and (c) a mixture enriched in constituent B and impoverished in constituent A is collected on the first side of said membrane,the process being characterised in that the thickness of the active layer is less than 1 micrometer and in that the ratio of the average size of particles of the dispersed adsorbent phase to the thickness of the active layer is less than 0.25.

2. Process according to claim 1 wherein the adsorbent phase dispersed in the continuous polymer comprises a molecular sieve type zeolite.

3. Process according to one of claims 1 to 2 wherein the thickness of the active layer is less than 0,1 micrometer.

4. Process according to one of claims 1 to 3 wherein the membrane is an asymmetric membrane obtained by contacting an antisolvent with the first face of a polymer film in solution in a solvent comprising the adsorbent phase dispersed under conditions such that said active layer resting in an undissociated manner on the underlying film which becomes porous and constitutes the support layers, is coagulated.

5. Process according to one of claims 1 to 4 wherein constituent A is hydrogen and constituent B is methane and wherein the dispersed adsorbent phase consists of a type 4A molecular sieve.

6. Process according to one of claims 1 to 5 wherein constituent A is methane and constituent B is water and wherein the dispersed adsorbent phase consists of a type 3A molecular sieve.

7. Process according to one of claims 1 to 6 wherein said mixture comprises n-paraffins and wherein the active layer comprises a type 5A molecular sieve, wherein the following combination is carried out:
a) hydrogen in the vapor phase and the mixture are introduced into an isomerization zone, under isomerization conditions such that n-paraffins, isoparaffins and hydrogen are collected;
b) the product in the vapor phase collected in this way is contacted with a first part of a separation zone, upstream from the membrane, and separation of n-paraffins, iso-paraffins and hydrogen is carried out by circulating, in a second part of said zone downstream from the membrane, at least a part of the hydrogen resulting from step c) below;
c) at the outlet of said first part, a second mixture of hydrogen and isoparaffins is collected, said second mixture is condensed and a fraction containing mainly isoparaffins on the one hand and hydrogen on the other is collected, which is recycled in the second part of the separation zone according to step b);
d) at the oulet of the second part of the separation zone, a third mixture rich in n-paraffins containing hydrogen is collected; and
e) at least part of the third mixture is recycled in the isomerization zone according to step a).

8. Process according to one of claims 1 to 7 wherein the non elastomeric polymer is a polyimide.

9. Process according to one of the claims 1 to 8 wherein the radio of the mass of dispersed phase to the mass of continuous phase in the active layer of the membrane is from 2 : 1 to 6 : 1.

## Patentansprüche

1. Verfahren zum Trennen eines Gemisches aus wenigstens zwei Bestandteilen A und B, bei dem man (a) dieses Gemisch in gasförmiger Phase oder (Wasser)dampf mit einer ersten Fläche einer Membran kontaktiert, die zusammengesetzt ist aus (i) wenigstens einer aktiven Schicht, die eine feste adsorbierende Phase, selektiv gegenüber dem Bestandteil A, dispergiert in einem nicht porösen, nicht elastomeren Polymer, umfaßt und eine kontinuierliche Phase bildet und (ii) einer porösen Schicht, die als Träger dient, der direkt an dieser aktiven Schicht haftet, wobei das Verhältnis der Masse dispergierter Phase zu der Masse kontinuierlicher Phase in der aktiven Schicht der Membran größer als 1 ist, (b) man einen partiellen Druck des Bestandteils A, der geringer auf der zweiten Fläche der Membran als auf der ersten Fläche der Membran ist, aufrechterhält und auf der zweiten Fläche dieser Membran ohne Phasenänderung ein am Bestandteil A angereichertes und am Bestandteil B verarmtes Gemisch sammelt und (c) man auf der ersten Fläche der Membran ein am Bestandteil B angereichertes und am Bestandteil A verarmtes Gemisch sammelt, wobei das Verfahren sich dadurch auszeichnet, daß die Dicke der aktiven Schicht kleiner als 1 Mikrometer ist und daß das Verhältnis der mittleren Abmessung der Partikel adsorbierender dispergierter Phase zur Dicke der aktiven Schicht kleiner als 0,25 ist.

2. Verfahren nach Anspruch 1, dadurch gekennzeichnet, daß die adsorbierende im Polymer in kontinuierlicher Phase dispergierte Phase gebildet wird durch einen Zeolith vom Typ Molekularsieb.

3. Verfahren nach einem der Ansprüche 1 bis 2, bei dem die mittlere Abmessung der Partikel kleiner als 0,1 Mikrometer ist.

4. Verfahren nach einem der Ansprüche 1 bis 3, bei dem die Membran eine asymmetrische Membran ist, die durch Kontaktierung eines Antisolvents auf der ersten Fläche eines Films aus Polymer in Lösung in einem Lösungsmittel erhalten wurde, das die adsorbierende Phase umfaßt, die unter Bedingungen derart dispergiert ist, daß man diese aktive Phase koagulieren läßt, die sich in undissoziierter Weise auf dem darunter angrenzenden porös gewordenen Film, der die Trägerschicht bildet, abstützt.

5. Verfahren nach einem der Ansprüche 1 bis 4, dadurch gekennzeichnet, daß der Bestandteil A Wasserstoff ist und der Bestandteil B Methan ist und daß die adsorbierende dispergierte Phase gebildet wird durch ein Molekularsieb vom Typ 4A.

6. Verfahren nach einem der Ansprüche 1 bis 5, dadurch gekennzeichnet, daß der Bestandteil A Methan und der Bestandteil B Wasser ist und daß die adsorbierende dispergierte Phase gebildet wird durch ein Molekularsieb vom Typ 3A.

7. Verfahren nach einem der Ansprüche 1 bis 6, bei dem das Gemisch n-Paraffine umfaßt und bei dem die aktive Schicht ein Molekularsieb vom Typ 5A umfaßt, dadurch gekennzeichnet, daß man die folgende Kombination vornimmt:
a) man gibt in dampfförmiger Phase Wasserstoff sowie das Gemisch in eine Isomerisierungszone unter Isomerisierungsbedingungen derart, daß man n-Paraffine, Isoparaffine und Wasserstoff sammelt;
b) man kontaktiert in dampfförmiger Phase das so gesammelte Produkt in einem ersten Teil einer Trennzone vor der Membran und man nimmt die Trennung der n-Paraffine, der Isoparaffine und des Wasserstoffs vor, indem man in einem zweiten Teil dieser Zone hinter der Membran wenigstens zum Teil Wasserstoff zirkulieren läßt, der aus der unten stehenden Stufe c) stammt;
c) man gewinnt am Ausgang dieses ersten Teils ein zweites Gemisch aus Wasserstoff und Isoparaffinen, man kondensiert dieses zweite Gemisch und man sammelt einerseits eine Fraktion, die im wesentlichen nur Isoparaffine enthält und andererseits Wasserstoff, den man entsprechend Stufe b) in den zweiten Teil der Trennzone recyclisiert;
d) man gewinnt am Ausgang des zweiten Teils der Trennzone ein drittes an n-Paraffinen angereichertes Gemisch, welches Wasserstoff enthält; und
e) man recyclisiert wenigstens zum Teil das dritte Gemisch in die Isomerisierungsstufe gemäß Stufe a).

8. Verfahren nach einem der Ansprüche 1 bis 7, bei dem das nicht elastomere Polymer eine Polyimid ist.

9. Verfahren nach einem der Ansprüche 1 bis 8, bei dem das Verhältnis der Masse an dispergierter Phase zur Masse an kontinuierlicher Phase in der aktiven Schicht der Membran 2 : 1 bis 6 : 1 beträgt.
